# FASCICULE DE BREVET EUROPEEN

(11) **EP 1 326 621 B1**
(45) Date de publication et mention de la délivrance du brevet: **02.03.2005**
(21) Numéro de dépôt: 01975890.3
(22) Date de dépôt: 16.10.2001
(51) Int. Cl.: A61K 33/14, A61K 33/42

(54) **COMPOSITION MEDICAMENTEUSE ET EN PARTICULIER SON UTILISATION DANS LA FLUIDOTHERAPIE**
MEDIZINISCHE ZUSAMMENSETZUNG UND INSBESONDERE SEINE VERWENDUNG IN DER FLÜSSIGKEITSTHERAPIE
MEDICINAL COMPOSITION AND IN PARTICULAR ITS USE IN FLUID THERAPY

(30) Priorité: 16.10.2000 EP 00203619
(43) Date de publication de la demande: 16.07.2003
(73) Titulaire: Université de Liège, 4000 Liège (BE); UNIVERSITE CATHOLIQUE DE LOUVAIN, 1348 Louvain-la-Neuve (BE)
(72) Inventeur: GUSTIN, Pascal, B-1150 Bruxelles (BE); CAMBIER, Carole, B-4000 Liège (BE); CLERBAUX, Thierry, Unté Catholique de Louvain, B-1200 (BE); FRANS, Albert, Unté Catholique de Louvain, B-1200 (BE); DETRY, Bruno, B-1460 Ittre (BE)
(74) Mandataire: Claeys, Pierre
(86) Numéro de dépôt international: PCT/BE2001/000180
(87) Numéro de publication internationale: WO 2002/032393

(56) Documents cités:
- DE-C- 3 820 840
- FR-A- 2 645 746
- US-A- 5 811 463

## Description

La présente invention est relative à une nouvelle composition médicamenteuse, et en particulier à son utilisation dans la fluidothérapie.

Chez les bovins, les pathologies toxi-infectieuses, parmi lesquelles la diarrhée néonatale et le choc endotoxinique, nécessitent un tel traitement.

En ce qui concerne la diarrhée néonatale, il est communément admis que le premier traitement à instaurer est la fluidothérapie orale ou parentérale, en fonction de l'état clinique de l'animal. Cependant, les thérapeutiques actuelles visent uniquement à rétablir l'équilibre hydrique et ionique, à rétablir le pH sanguin et à couvrir un déficit énergétique éventuel. Jusqu'ici, le traitement habituellement conseillé dans la littérature scientifique traitant de la fluidothérapie parentérale chez le bovin repose sur l'administration de larges volumes de solutions cristalloïdes isotoniques, les quantités perfusées étant calculées en fonction notamment du degré de déshydratation et d'acidose de l'animal. Afin de rétablir le volume circulant, d'autres auteurs préconisent également l'administration de petits volumes d'une solution saline hypertonique, additionnée ou non de dextran. Selon la littérature, ces solutions salines doivent être accompagnées de l'administration d'un agent alcalinisant afin de rétablir le pH sanguin. Il apparaît donc qu'à ce jour, les troubles de l'oxygénation tissulaire existant chez le veau diarrhéique ne sont jamais pris en compte dans l'évaluation de l'efficacité du traitement. Plus grave, dans certains cas, les thérapeutiques actuelles peuvent même aggraver ces troubles de l'oxygénation tissulaire. En effet, par exemple, l'administration massive de bicarbonates en vue de rétablir le pH peut causer une alcalose relative et une hypochlorémie, favorisant ainsi l'augmentation de l'affinité de l'hémoglobine pour l'oxygène et rendant le transport de l'oxygène par le sang moins efficient.

En ce qui concerne le choc endotoxinique, il est également admis que l'instauration d'un traitement par fluidothérapie parentérale est une priorité. Cependant, celui-ci vise essentiellement à rétablir le volume circulant. La littérature scientifique décrit l'utilisation de différentes solutions de perfusion, à savoir : les solutés cristalloïdes isotoniques, les solutés cristalloïdes hypertoniques (additionnés ou non d'un colloïde comme le dextran), mais aussi le plasma et le sang total. Les effets de ces différentes thérapeutiques sur le transport de l'oxygène par l'hémoglobine et l'oxygénation des tissus n'ont toutefois pas été étudiés de manière approfondie.

Par ailleurs, les brevets US 5.238.684, 5.236.712, 5.147.650, 5.089.477 et 4.981.687 décrivent des formulations destinées à être administrées de préférence par voie orale, qui visent à augmenter la réponse physiologique à l'exercice, notamment via une augmentation du débit cardiaque. Ces solutions contiennent de l'eau, des électrolytes, du glycérol et une source d'énergie additionnelle. Ces solutions ne visent aucunement à manipuler le transport de l'oxygène par l'hémoglobine, ni à améliorer l'oxygénation tissulaire.

On cônnaît, pour le traitement des troubles de l'ischémie-reperfusion, une composition médicamenteuse comprenant, entre autres, du chlorure de sodium hypertonique et du phosphate monopotassique (v. DE-C-3820840). Cette composition doit contenir en outre une série d'autres composants dont obligatoirement un antagoniste du calcium, substance connue pour son effet négatif sur le débit cardiaque, et pour sa tendance à provoquer une dépression des contractions du coeur et une hypotension.

La présente invention a pour but de mettre au point une nouvelle composition médicamenteuse. Celle-ci doit pouvoir lutter contre l'hypoxie tissulaire qui survient au cours de pathologies toxi-infectieuses, chez les mammifères, en particulier chez les ruminants, tels que les bovins, et y compris les êtres humains. Au cours de ces processus pathologiques, l'invention doit permettre de maintenir chez les êtres atteints leur consommation en oxygène grâce, notamment, à une augmentation de la quantité d'oxygène extraite au niveau des tissus, tout en combinant ce mécanisme original avec une augmentation du débit cardiaque.

Pour résoudre ce problème, il est prévu, suivant l'invention, une utilisation d'une composition pour la fabrication d'un médicament destiné à simultanément augmenter la quantité d'oxygène extraite au niveau des tissus et augmenter le débit cardiaque chez des mammifères atteints de troubles hypoxiques des tissus, en particulier de ceux survenant au cours de pathologies toxiinfectieuses, cette composition comprenant, en quantités thérapeutiquement efficaces, du chlorure de sodium hypertonique, au moins une molécule exerçant de manière directe ou indirecte un effet de relaxation des muscles lisses vasculaires, et au moins une molécule susceptible de fournir un apport exogène en phosphates.

Cette composition comprend un "trépied" thérapeutique nécessaire pour garantir le succès thérapeutique visé. Elle agit par une combinaison de mécanismes d'action originaux permettant une meilleure extraction de l'oxygène au niveau tissulaire, à savoir une diminution de l'affinité intrinsèque de l'hémoglobine pour l'oxygène, une augmentation in vivo du transport de l'oxygène par l'hémoglobine et une augmentation de la capacité intrinsèque des tissus à extraire l'oxygène. La meilleure extraction de l'oxygène au niveau tissulaire évite le développement d'une hypoxie tissutaire.

Suivant une forme de réalisation de l'utilisation suivant l'invention, la composition comprend en outre un agent alcalinisant susceptible d'augmenter un pH sanguin dans une gamme compatible avec un transport d'oxygène adéquat. Cet agent alcalinisant en quantité adéquate permet un maintien de l'acidose relative. Comme agents alcalinisants on peut mentionner entre autres des bicarbonates, des acétates, des propionates et des lactates, en particulier du bicarbonate de sodium.

Suivant une forme avantageuse de l'utilisation suivant l'invention, la composition consiste en une combinaison d'une première solution de perfusion et d'une deuxième solution de perfusion, la première solution de perfusion comprenant de l'eau, ledit chlorure de sodium hypertonique et ladite au moins une molécule exerçant un effet de relaxation, et la deuxième solution de perfusion comprenant de l'eau, et ladite au moins une molécule susceptible de fournir un apport exogène en phosphates. Grâce à cette forme de réalisation, administrée par perfusion, par exemple par voie intraveineuse, il est possible d'augmenter le débit cardiaque ce qui renforce le phénomène d'extraction de l'oxygène au niveau tissulaire.

Une telle formulation permet une fabrication et un stockage séparés des composants ainsi qu'éventuellement une administration chronologiquement séparée des deux solutions, en fonction de la vitesse d'action des composants envisagés. Elle permet d'ailleurs aussi, si nécessaire, une perfusion simultanée des deux solutions à deux endroits différents du corps à traiter et une application étalée dans le temps de ces solutions.

Suivant l'invention, le chlorure de sodium est à une concentration de 5 à 10 % dans ladite première solution de perfusion. Avantageusement, on fera usage de doses d'environ 5 à 10 ml/kg de poids de corps. Par molécule exerçant un effet de relaxation des muscles lisses on peut entendre, suivant l'invention, les vasodilatateurs en général, et la caféine en particulier. Par molécule susceptible de fournir un apport exogène en phosphates, on peut entendre suivant l'invention un phosphate inorganique, par exemple du phosphate de sodium, ou un phosphate organique, tel qu'un triphosphate, par exemple du triphosphate d'adénosine, ou du diphosphoglycérate, ainsi qu'en général toute substance capable de modifier la teneur intra-érythrocytaire en diphosphoglycérate.

Suivant une forme particulière de l'utilisation suivant l'invention, la deuxième solution de perfusion contient en outre au moins un composant destiné à corriger l'équilibre hydrique, électrolytique et énergétique. Par composant de ce genre, on peut entendre du chlorure de sodium, du chlorure de potassium, du glucose, etc.

D'autres utilisations suivant l'invention sont indiquées dans les revendications annexées.

La présente invention concerne également une composition médicamenteuse pour la mise en oeuvre d'un traitement thérapeutique ou prophylactique de troubles hypoxiques des tissus, en particulier de ceux survenant au cours de pathologies toxi-infectieuses chez les mammifères, consistant en une combinaison d'une première solution de perfusion et d'une deuxième solution de perfusion,
la première solution de perfusion comprenant de l'eau, du chlorure de sodium hypertonique et au moins une molécule exerçant un effet de relaxation, et
la deuxième solution de perfusion comprenant de l'eau, et au moins une molécule susceptible de fournir un apport exogène en phosphates.

D'autres compositions médicamenteuses suivant l'invention sont indiquées dans les revendications données dans la suite, ainsi que l'utilisation de ces compositions pour la fabrication d'un médicament destiné au traitement thérapeutique ou prophylactique de troubles hypoxiques des tissus, en particulier de ceux survenant au cours de pathologies toxi-infectieuses, chez les mammifères.

L'invention va à présent être décrite de manière détaillée à l'aide d'exemples donnés ci-dessous à titre non limitatif.

### Exemple 1

Dans les conditions normales, le transport de l'oxygène sous forme dissoute ne représente qu'une faible proportion (environ 1 %) du transport total. Pour le reste, l'oxygène est acheminé vers les tissus après fixation sur l'hémoglobine.

Au niveau pulmonaire, l'hémoglobine se sature en oxygène. Dans les tissus au repos, environ un tiers de la quantité totale ainsi fixée diffuse dans le liquide interstitiel, le reste retournant vers les poumons. La fixation de l'oxygène sur l'hémoglobine dépend avant tout de la pression partielle en oxygène (PO₂) dans le plasma.

Classiquement, chez tous les mammifères, les facteurs régulateurs de l'affinité de l'oxygène pour l'hémoglobine sont le pH sanguin, la température corporelle, la pression partielle en dioxyde de carbone, le taux de fixation de 2,3-diphosphoglycérate (2,3 DPG) sur l'hémoglobine.

Ces mécanismes permettent les échanges de l'oxygène dans l'organisme. Dans le sang artériel, la pression partielle en oxygène est élevée, la température et les teneurs en CO₂ diminuent, tandis que le pH augmente, autant de conditions favorables à la fixation de l'oxygène sur l'hémoglobine. Dans le sang veineux et dans les tissus, au contraire, la pression partielle en oxygène est faible, le pH diminue, la température et les teneurs en CO₂ augmentent, autant de facteurs favorables à la libération de l'oxygène.

Lors d'une étude ayant pour but d'investiguer les troubles de l'oxygénation tissulaire chez le veau diarrhéique, en considérant l'organisme non plus dans sa globalité, mais dans un territoire vital corporel en particulier, il a pu être démontré qu'une adaptation de cette oxygénation par une augmentation de l'extraction de l'oxygène au niveau tissulaire n'existait pas dans tous les organes. La capacité des tissus à extraire l'oxygène est même apparue diminuée. Il est apparu que, si l'on considère les échanges se produisant entre le sang artériel et le sang veineux jugulaire, les veaux diarrhéiques souffrent d'une incapacité à extraire l'oxygène qui leur est apporté par le sang. Cette incapacité peut être expliquée par :
- une augmentation de l'affinité de l'oxygène pour l'hémoglobine, illustrée par la diminution de la pression partielle en oxygène à 50 % de saturation de l'hémoglobine mesurée dans des conditions standards (pH : 7,4; PCO₂ : 40 mm de Hg; température : 37°C), appelée ci-après P50 standard (P50 std). Ce phénomène s'explique notamment par une diminution du taux de 2,3 DPG;
- une hypothermie et une hypocapnie, qui s'opposent aux effets positifs de l'acidose sur le transport de l'oxygène par le sang, ainsi que l'illustre la chute des pressions partielles en oxygène à 50 % de saturation de l'hémoglobine dans les compartiments artériel (P50a) et veineux (P50v);
- une incapacité intrinsèque des tissus à extraire l'oxygène qui leur est apporté, ainsi que l'illustre l'augmentation de la pression partielle en oxygène au niveau veineux (PvO₂) chez les veaux diarrhéiques.

On a par conséquent procédé dans le présent exemple à une série de traitements de veaux diarrhéiques par une méthode classique, par un produit du commerce, par différents composants d'une composition suivant l'invention et par une composition suivant l'invention, dans des buts de comparaison.

Dans tous les exemples ci-dessous, les dosages sont calculés par rapport à 1 kg de poids de corps.
a) La solution classique testée est une solution cristalloïde isotonique de chlorure de sodium (40 ml/kg), de glucose (20 ml/kg) et de bicarbonate de sodium, à perfuser. La quantité de bicarbonate de sodium à apporter est calculée comme suit : nombre de mmoles de bicarbonate à apporter = Poids (kg) x déficit en base dans le sang veineux (mmoles/l) x 0,6 (l/kg), où 0,6 représente le volume de distribution du bicarbonate dans le compartiment liquidien extracellulaire. Vitesse de perfusion: 50 ml/kg/h la première heure, puis 20 ml/kg/h.
b) La solution commerciale, le Lactétrol^{R}, est un médicament indiqué pour la correction des déshydratations, accompagnées ou non d'acidose métabolique, chez les bovins. Il est important de noter que ce produit est un des rares médicaments disponibles sur le marché pour le traitement, par fluidothérapie parentérale, de symptômes associés à la diarrhée néonatale. La formule de la spécialité est la suivante :

| | |
|---|---|
| Chlorure de sodium | 5,76 mg |
| Chlorure de potassium | 0,37 mg |
| Chlorure de calcium | 0,37 mg |
| Chlorure de magnésium | 0,2 mg |
| Lactate de sodium | 5,04 mg |
| Parahydroxybenzoate de méthyle | |
| Eau pour injection | ad 1 ml. |

Volume à administrer : 40 ml/kg. Vitesse de perfusion : 10 ml/kg/h.
c) Une composition constituée de deux solutions de perfusion :
- solution A hypertonique (NaCl 7,5 %, 5 ml/kg, 1 ml/kg/min) : cette solution étant à la limite de l'osmolarité permise en thérapeutique, les éléments visant à rétablir l'équilibre ionique et à maintenir l'équilibre hydrique et le pH sont amenés via une solution B, administrée séparément.
- solution B isotonique (glucose : 20 ml/kg et bicarbonate de sodium administré en quantité suffisante pour ramener le déficit en base à zéro). Cette solution peut éventuellement être complétée par d'autres ions (K⁺, Mg⁺⁺ ...). Vitesse d'administration : 50 ml/kg/h la première heure, puis 20 ml/kg/h.
La quantité de bicarbonate de sodium à apporter est calculée comme précédemment décrit.
d) Une composition constituée de deux solutions de perfusion :
solution A hypertonique (NaCl 7,5 %, 7,5 ml/kg additionnée de 0,4 g de caféine par litre; 1 ml/kg/min) : cette solution étant à la limite de l'osmolarité permise en thérapeutique, les éléments visant à rétablir l'équilibre ionique et à maintenir l'équilibre hydrique et le pH sont amenés séparément via une solution B.
solution B (NaCl isotonique + glucose: 10 g/l + chlorure de potassium : 1 g/l : 40 ml/kg et bicarbonate de sodium isotonique administré en quantité suffisante pour ramener le pH à 7,2). Cette solution peut éventuellement être complétée par d'autres ions (Mg⁺⁺...). Vitesse d'administration : 20 ml/kg/h.
La quantité de bicarbonate de sodium à apporter a été calculée selon l'équation Henderson-Hasselbalch.
e) Une composition constituée comme suit :
chlorure de sodium isotonique + glucose: 10 g/l + chlorure de potassium : 1 g/l : 40 ml/kg, et phosphates inorganiques isotoniques (NaH₂PO₄.H₂O : 2,45 g/l, Na₂HPO₄.2H₂O: 19,82 g/l) : 16 ml/kg. Cette solution peut éventuellement être complétée par d'autres ions (Mg⁺⁺...). Vitesse d'administration : 27,5 ml/kg/h.
f) Composition suivant l'invention constituée de deux solutions de perfusion :
   solution A :
      Eau stérile apyrogène
      NaCl hypertonique (7,5 %) : 75 g/l
      Caféine : 0,4 g/l
      (7,5 ml/kg; vitesse de perfusion : 1 ml/kg/min)
   solution B :
      Eau stérile apyrogène.
      Solution isotonique tampon de phosphates inorganiques (NaH₂PO₄.H₂O : 2,45 g/l, Na₂HPO₄.2H₂O: 19,82 g/l) : 16 ml/kg;
      vitesse de perfusion : 25 ml/kg/h.
En vue de rétablir la volémie, de corriger l'équilibre électrolytique et de maintenir le pH dans des limites compatibles avec le transport de l'oxygène (7,2), divers composés peuvent être ajoutés à cette solution, à savoir : NaCl, KCI, MgCl₂, glucose, NaHCO₃.

Par exemple: solution de chlorure de sodium isotonique + glucose 10 g/l + chlorure de potassium 1 g/l (40 ml/kg) et bicarbonate de sodium isotonique si nécessaire (pH < 7,2).

Chacune de ces compositions a été testée sur un échantillon d'environ 10 veaux diarrhéiques. Il faut noter que dans le cas des compositions formées de deux solutions, la solution B a été perfusée 15 minutes après le début de la perfusion de la solution A.

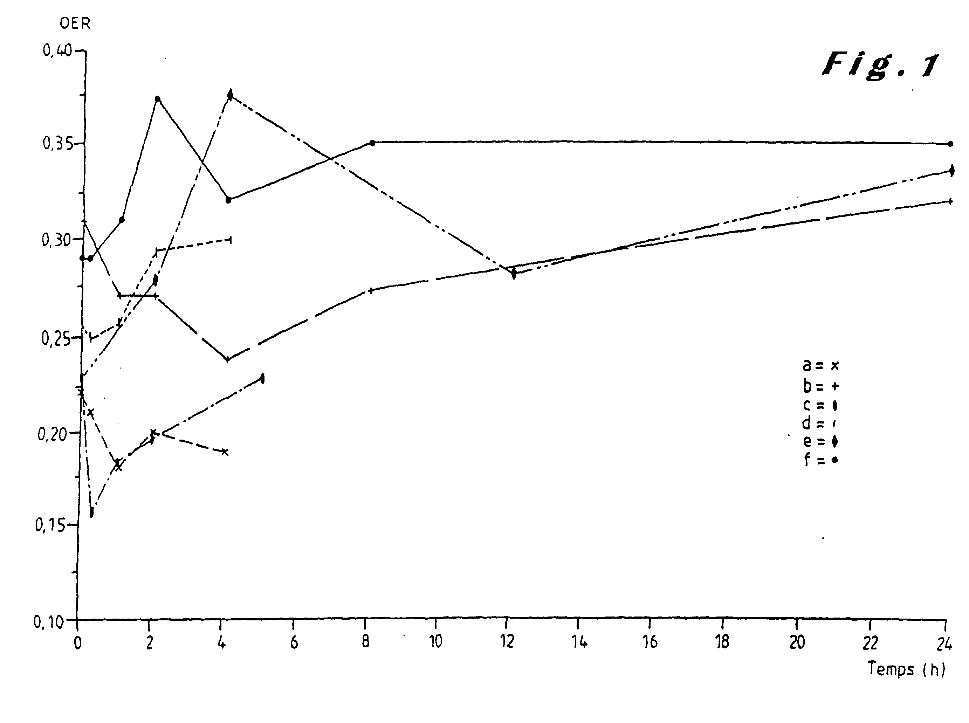

La figure 1 illustre l'évolution en fonction du temps de la quantité d'oxygène extraite au niveau tissulaire (OER) suite à l'administration de chacune des compositions a) à f). OER est le rapport entre la différence du contenu en oxygène dans le sang artériel (CaO₂) et veineux (CvO₂) et le contenu en oxygène dans le sang artériel. Pratiquement, le contenu artériel et le contenu veineux en oxygène sont calculés comme suit :

CaO₂ = Hb x BO₂ x [SaO₂/100] + (α x PaO₂)

CvO₂ = Hb x BO₂ x [SvO₂/100] + (α x PvO₂)

où Hb représente le taux d'hémoglobine de l'animal, BO₂ représente la quantité d'oxygène fixée par l'hémoglobine (1,39 mlO₂/g Hb) et α représente le coefficient de solubilité de l'oxygène (0,003 ml.100ml⁻¹ . mm Hg⁻¹).

La fraction d'oxygène extraite au niveau tissulaire s'exprime alors comme un rapport : OER = (CaO₂ - CvO₂)/CaO₂

De cette figure 1 on peut déduire que le traitement classique a) et la solution Lactétrol b) ne sont pas capables d'augmenter l'extraction de l'oxygène au niveau tissulaire.

Alors que l'on avait remarqué un effet favorable de l'ion chlore sur le transport de l'oxygène in vivo, chez des veaux sains (Cambier et al, Effects of hyperchloremia on blood oxygen binding in healthy calves, The American Physiological Society, 1998, p. 1267-1272), ce traitement (composition c)) s'est malheureusement révélé incapable d'augmenter l'extraction de l'oxygène au niveau tissulaire des veaux diarrhéiques, ce paramètre ayant même tendance à diminuer. Les résultats obtenus, in vivo, chez le veau sain, ne sont donc pas transposables au veau malade.

Enfin, il apparaît avec la composition d) une légère augmentation de la quantité d'oxygène extraite, qui toutefois ne s'avère pas significative, tandis que la composition e) et la composition suivant l'invention f) présentent une augmentation significative et durable de celle-ci.

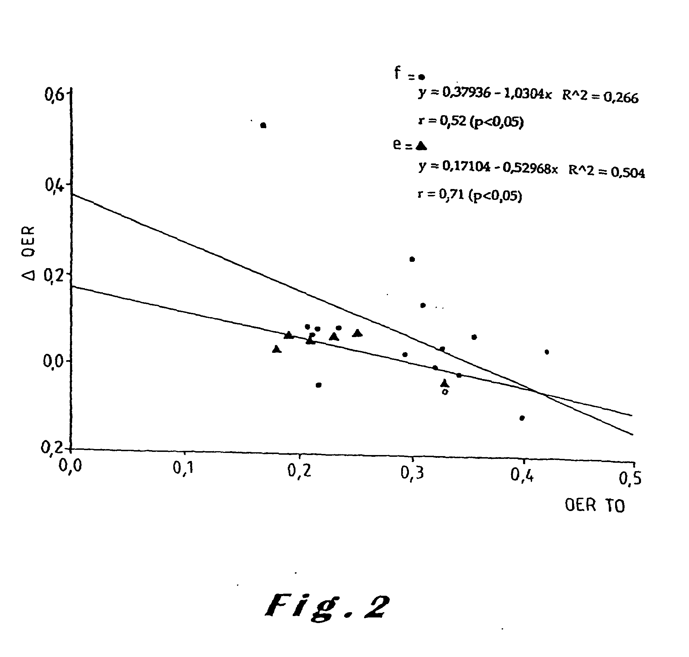

Cependant, l'avantage de la composition suivant l'invention f) par rapport à la composition e) apparaît lorsque les variations de l'OER enregistrées en cours de traitement sont mises en relation avec les valeurs initiales d'OER mesurées à T0 (figure 2). Une corrélation négative significative est observée avec les deux traitements. Ceci signifie que les animaux présentant les déficiences les plus marquées du point de vue de l'OER tirent le plus grand bénéfice des thérapies. Toutefois, comparant les deux solutions e) et f), il apparaît que les augmentations d'OER obtenues avec la composition e) sont inférieures à celles obtenues avec la composition suivant l'invention f), ainsi qu'en attestent les ordonnées à l'origine des équations des régressions linéaires relatives à ces deux traitements (respectivement 0,17 pour la composition e) et 0,38 pour la composition suivant l'invention f).

En outre, il faut souligner que seule la composition suivant l'invention f) met en jeu tous les mécanismes permettant une augmentation de l'extraction de l'oxygène au niveau tissulaire. Ce phénomène peut se révéler très important en conditions pathologiques, un mécanisme pouvant suppléer l'autre en cas de déficience.

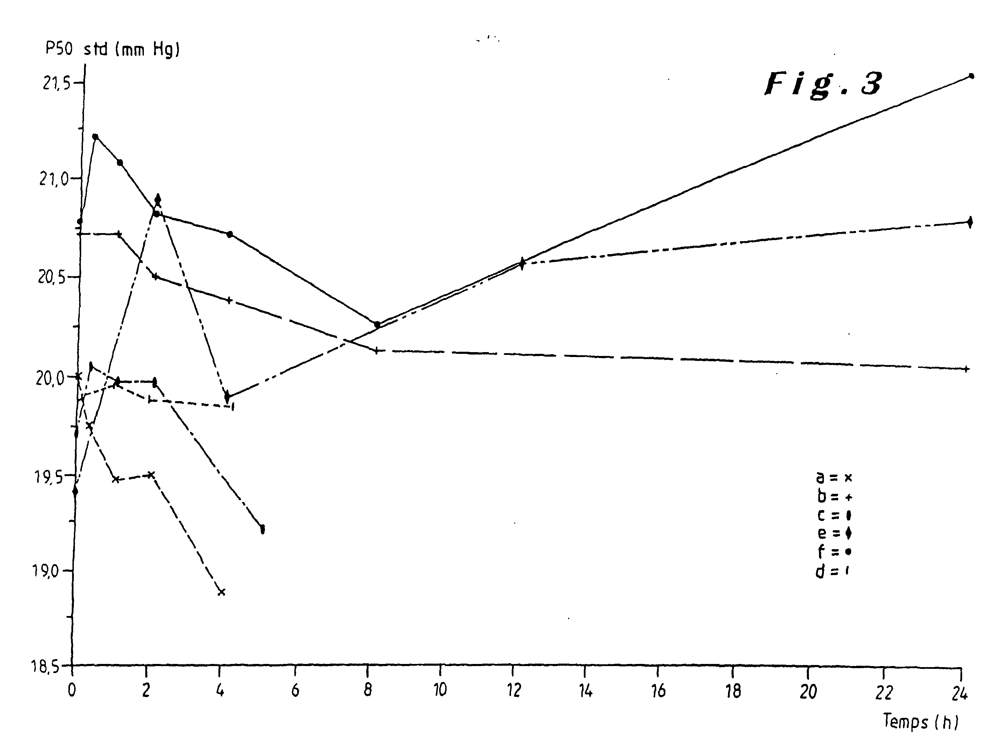

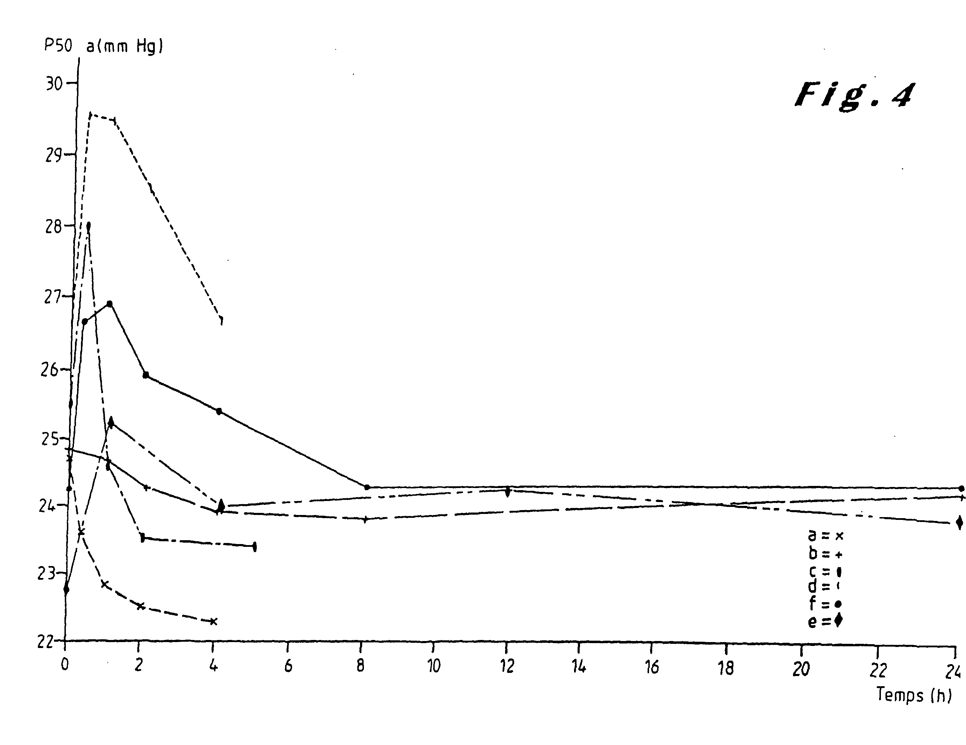

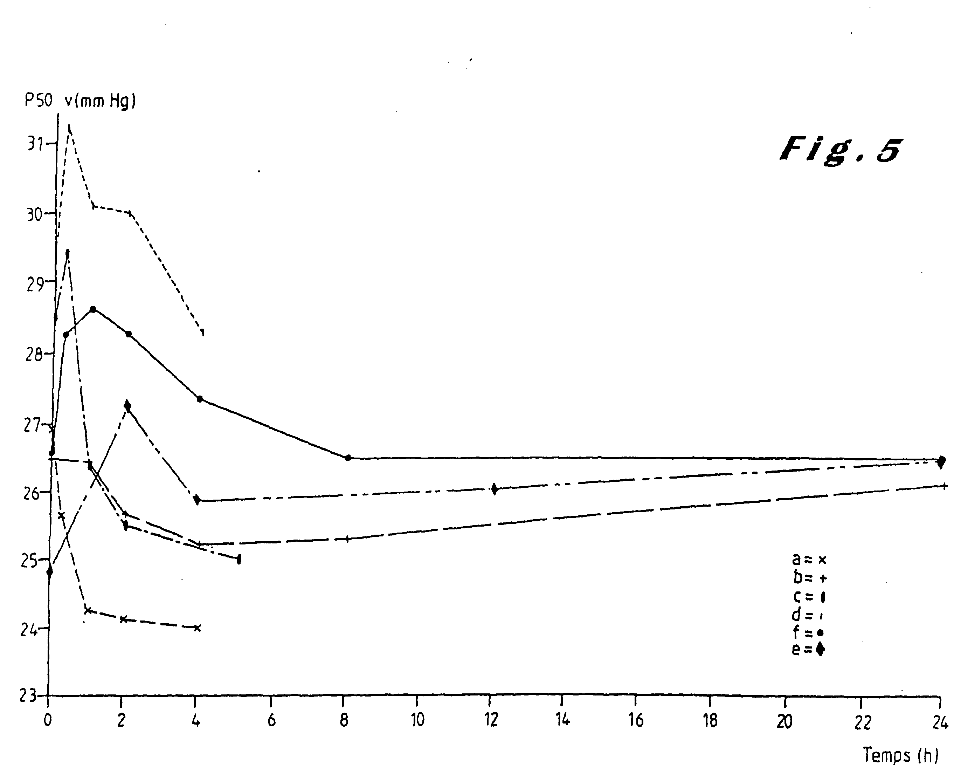

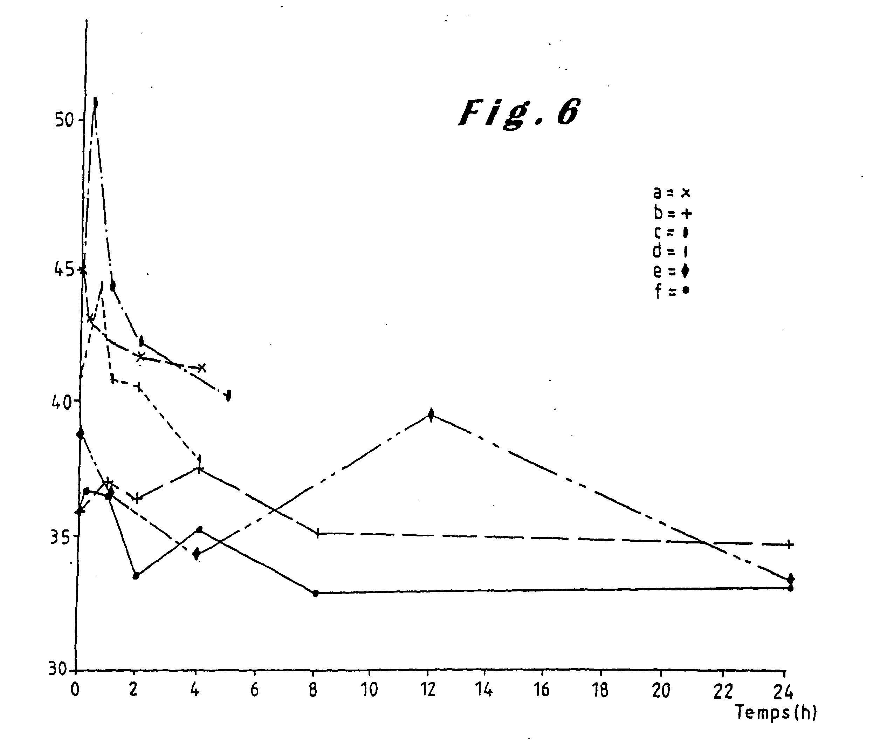

Pour rappel, l'extraction de l'oxygène au niveau tissulaire peut être améliorée via trois mécanismes :
1. par une diminution de l'affinité intrinsèque de l'hémoglobine pour l'oxygène. Ce phénomène est illustré par une augmentation de la P50 std. La figure 3 montre une augmentation significative de la P50 std lors de l'administration de la composition e) et lors de l'administration de la composition suivant l'invention f) alors qu'aucun effet significatif n'est obtenu, ou même que des effets délétères (diminution de la P50 standard) sont enregistrés, avec les autres traitements.
2. par une modification, in vivo, des facteurs de contrôle du transport de l'oxygène par le sang, et notamment le pH. Les effets bénéfiques enregistrés, in vitro, sur la P50 std (cf point 1) peuvent ainsi être augmentés ou diminués chez l'animal. In vivo, la capacité de transport de l'oxygène par le sang est évaluée en calculant les pressions partielles en oxygène P50 a et P50 v. Idéalement, une augmentation de la P50 a et de la P50 v est souhaitée, en renforçant, via notamment des variations de pH, les effets sur la P50 std.
   Comme l'illustrent les figures 4 et 5, in vivo, la composition e) et la composition suivant l'invention f), améliorent les capacités de transport de l'oxygène. Cependant, en ce qui concerne la composition e), cet effet trouve principalement son origine dans la diminution de l'affinité intrinsèque de l'hémoglobine pour l'oxygène, illustrée par l'augmentation de la P50 std. Ce phénomène n'est que peu renforcé in vivo. Ainsi, à t= 2 heures, si la composition e) augmente la P50 std de 8 %, les P50 a et P50 v ne sont augmentées que de, respectivement, 10 et 9 %, montrant l'absence d'intervention d'autres mécanismes favorables à la libération de l'oxygène au niveau tissulaire. In vivo, l'intervention de ces mécanismes régulateurs du transport de l'oxygène peut se révéler très importante chez des animaux dont la P50 standard n'augmente que faiblement suite à la perfusion. Ainsi les effets obtenus sur la P50 standard avec la composition e) sont variables. Pour preuve, à t = 2 heures, l'augmentation de P50 standard varie de 4 à 13 % selon les individus, et seuls 33 % des animaux traités avec la composition e) maintiennent une augmentation de P50 standard durant 24 heures. Chez ces animaux, le recours à d'autres mécanismes régulateurs serait nécessaire. Contrairement à la solution e), la composition suivant l'invention f) permet, outre l'augmentation de la P50 standard, le développement d'une hyperchlorémie et le maintien d'une acidose relative, deux éléments renforçant les effets sur la P50 standard et favorables à la libération de l'oxygène au niveau tissulaire. Le poids de ces divers mécanismes varie au cours du temps. Ainsi, à t= 1 heure, la composition suivant l'invention f) induit une augmentation de la P50 standard de seulement 1 % mais augmente les P50 a et P50 v, respectivement de 11 et de 8 %, du fait du développement d'une hyperchlorémie et du maintien d'une acidose relative. A t= 24 heures, la balance entre ces deux fonctions s'inverse, puisque le rôle joué par les modifications de l'affinité intrinsèque de l'hémoglobine pour l'oxygène devient prépondérant;
3. par une augmentation de la capacité des tissus à extraire l'oxygène, marquée par une diminution de la pression partielle en oxygène au niveau veineux (PvO₂).
   Ainsi que l'illustre la figure 6, la composition hypertonique c) augmente de manière significative la PvO₂. L'administration d'un vasodilatateur, simultanément à une solution hypertonique (composition d) ou l'absence de solution hypertonique, comme c'est le cas de la composition e), permet d'annuler cet effet. En combinant un vasodilatateur, une solution hypertonique de NaCl et les phosphates, selon la composition suivant l'invention f), la capacité propre des tissus à extraire l'oxygène est optimale et se maintient tout au long du traitement.

Les méthodes de détermination de la PvO₂ et des P50 sont décrites par exemple dans C. Cambier et al., American Journal of Veterinary Research, 61, 2000, p. 299-304.

Outre ces trois mécanismes intervenant dans l'augmentation de l'extraction de l'oxygène au niveau tissulaire, une solution de perfusion peut également induire des effets bénéfiques via des modifications hémodynamiques (augmentation du débit cardiaque).

Selon les données de la littérature, et sur base de notre propre expérience chez le veau atteint de choc endotoxinique (cf exemple 3), l'hypoxie peut résulter :
- d'une altération de l'apport d'oxygène par le sang (DO₂) consécutive, notamment, à des perturbations hémodynamiques (diminution du débit cardiaque),
- d'une diminution de l'extraction de l'oxygène au niveau tissulaire, qui vient d'être discutée.

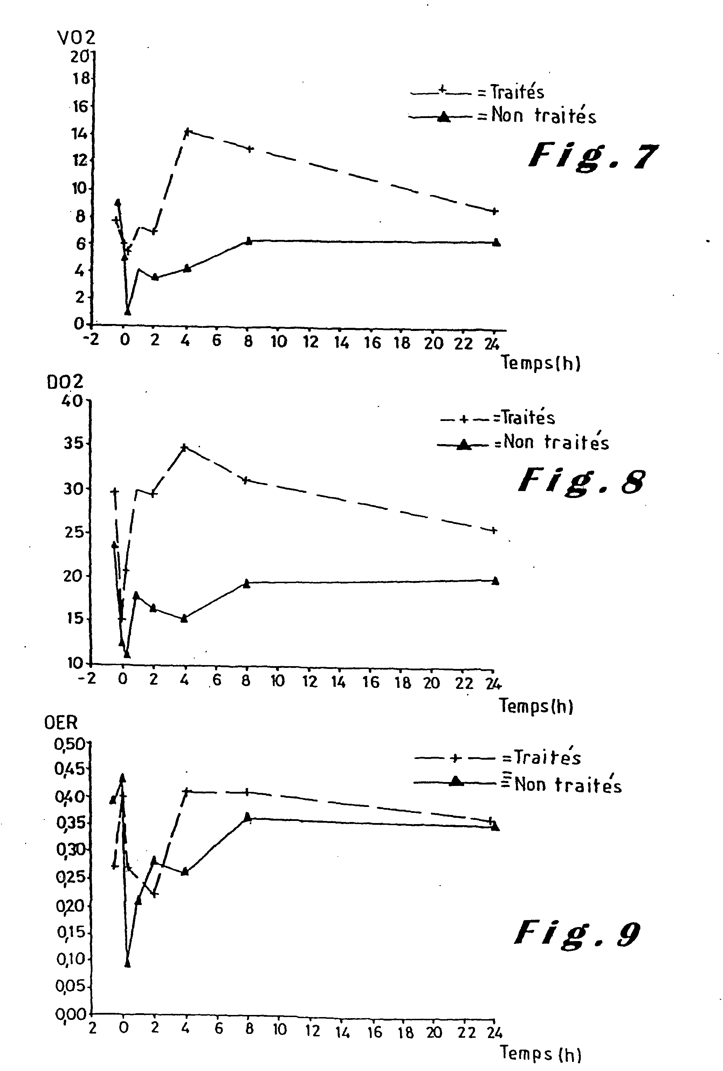

Ces deux mécanismes se combinent à des degrés divers chez les individus atteints de pathologies toxi-infectieuses pour aboutir aux état hypoxiques constatés cliniquement. Il est dès lors capital de disposer de solutions thérapeutiques permettant d'améliorer l'ensemble de ces fonctions, et ce par tous les mécanismes possibles. La solution suivant l'invention, outre ses effets sur l'OER, améliore la DO₂ du fait de l'augmentation du débit cardiaque liée à l'apport hydrique mais surtout à un effet hyperosmotique dans le compartiment intravasculaire. La figure 8 illustre l'augmentation de la DO₂ enregistrée chez des veaux souffrant de choc endotoxinique et traités avec la composition suivant l'invention.

L'administration d'une solution isotonique, comme la solution e), peut induire une augmentation du débit cardiaque du fait de l'apport hydrique mais cet effet reste limité et différé dans le temps par rapport aux effets quasiment immédiats et importants des solutions hypertoniques, comme la composition suivant l'invention f).

En résumé, outre le fait d'exercer un effet original plus important sur l'OER (cf figure 2) que la solution e), la solution suivant l'invention f) agit donc sur tous les mécanismes susceptibles de lutter contre les troubles de l'extraction de l'oxygène au niveau tissulaire.

De plus, elle exerce des effets hémodynamiques immédiats et importants de par ses effets directs mais aussi hyperosmotiques.

### Exemple 2

Un essai comparatif a été effectué sur trois échantillons de. veaux diarrhéiques. Le premier échantillon (n = 12) a été soumis au traitement classique a) décrit dans l'exemple 1, le deuxième échantillon (n = 18) au Lactétrol et le troisième échantillon (n = 16) à la composition suivant l'invention f) de l'exemple 1.

On a ensuite établi un score clinique des veaux traités en analysant les symptômes de la maladie, et en intégrant des évaluations telles que la diminution du réflexe de succion, le ralentissement du réflexe à la menace, la diminution de la sensibilité tactile, la diminution de la capacité à se tenir debout, etc... Le score est de 0 pour les veaux sains et de 13 chez un animal comateux. Les résultats de cette évaluation sont réunis dans le tableau 1 ci-dessous.

**Tableau 1**

| Paramètres/ Temps (h) | Invention (n=16) | Trt classique (n=12) | Trt lactétrol (n=18) |
|---|---|---|---|
| Score clinique | | | |
| 0 | 3,6 ± 0,6 | 5.1 ± 1,2 | 4,6 ± 0,8 |
| 0,25 | 3,3 ± 0,5 | 4,9 ± 1,2 | N.D. |
| 1 | 2,6 ± 0,4**†Δ | 4,9 ± 1,1 | 4,5 ± 0,8 |
| 2 | 1,7 ± 0, 3***†Δ | 4,3 ± 1,1 | 3,9 ± 0,7* |
| 4 | 1,3 ± 0,3***†ΔΔ | 3,3 ± 0,9* | 3,6 ± 0,8** |
| 8 | 0,9 ± 0,2**ΔΔ | N.D. | 2,9 ± 0,6** |
| 24 | 1,4 ± 0,3***Δ | N.D. | 3,6 ± 0,8* |

| | | | |
|---|---|---|---|
| N.D. : non déterminé. Trt : traitement Les valeurs sont exprimées comme suit : moyennes ± SE. * : valeur significativement différente de la valeur enregistrée à T0 dans le même groupe (* : p<0,05, ** : p<0,01, *** : p<0,001) | | | |
| † : valeur significativement différente de la valeur enregistrée au même moment dans le groupe traitement classique (†: p<0,05) | | | |
| Δ : valeur significativement différente de la valeur enregistrée au même moment dans le groupe traitement Iactétrol^{R} (Δ : p<0,05, ΔΔ : p<0,01). | | | |

### Exemple 3

Des troubles systémiques d'extraction de l'oxygène au niveau tissulaire ayant été mis en évidence lors de choc endotoxinique, un essai comparatif a été effectué sur deux échantillons de veaux sains ayant reçu une injection intraveineuse d'endotoxines d'Escherichia coli sérotype 055:B5, 0,2 µg/kg, au temps -30 minutes. Au temps 0, un premier échantillon (n = 3) est soumis au traitement d'une composition suivant l'invention et un deuxième échantillon (n = 3) n'est soumis à aucun traitement.

Ainsi que le montre la figure 7, contrairement à ce qui a été observé chez les animaux non traités, la composition suivant l'invention permet de maintenir la consommation en oxygène (VO₂) à son niveau de base, voire de l'augmenter, empêchant ainsi le développement d'une hypoxie tissulaire. Ce maintien, voire cette augmentation, sont rendus possibles grâce aux mécanismes suivants :
- un maintien de la quantité d'oxygène amenée aux tissus par le sang (DO₂), du fait notamment, de l'augmentation du débit cardiaque (effet hémodynamique). L'évolution de la DO₂ est reprise à la figure 8;
- une augmentation de la capacité d'extraction de l'oxygène au niveau tissulaire, illustrée à la figure 9.

Cette augmentation de l'OER s'explique par les mêmes phénomènes que ceux décrits chez les veaux diarrhéiques.

### Exemple 4

Un essai comparatif a été effectué selon la méthodologie décrite pour l'exemple 3. Au cours de cet essai, un score clinique a été établi selon les critères décrits pour l'exemple 2.

Les résultats de cette évaluation sont réunis dans le tableau 2 ci-dessous.

**Tableau 2**

| Paramètres/ Temps (h) | Veaux traités (n=3) | Veaux non traités (n=3) |
|---|---|---|
| Score clinique | | |
| -0,5 | 1,0 ± 0,0 | 1,0 ± 0,0 |
| 0 | 4,3 ± 2,0 | 4,0 ± 0,6* |
| 0,25 | 4,7 ± 1,7* | 7,0 ± 0,3**Δ |
| 1 | 4,7 ± 1,7 | 7,7 ± 0,9*Δ |
| 2 | 3,8 ± 1,6 | 6,0 ± 0,6* |
| 4 | 2,5 ± 0,5* | 3,3 ± 0,3* |
| 8 | 1,17 ± 0,2 | 1,7 ± 0,7 |
| 24 | 1,0 ± 0,0 | 1,7 ± 0,7 |

| | | |
|---|---|---|
| Les valeurs sont exprimées comme suit : moyennes ± SE. * : valeur significativement différente de la valeur enregistrée à T-30 (valeur physiologique) dans le même groupe (* : p<0,05, ** : p<0,01) | | |
| Δ : valeur significativement différente de la valeur enregistrée T0 dans le même groupe (Δ : p<0,05). | | |

Il doit être entendu que la présente invention n'est en aucune façon limitée aux formes de réalisation décrites ci-dessus et que bien des modifications peuvent y être apportées sans sortir du cadre des revendications annexées.

On peut par exemple envisager une composition sous la forme d'au moins une formulation solide, par exemple une poudre ou un comprimé soluble ou effervescent, destinée à la constitution d'au moins une solution aqueuse de perfusion par mélange avec de l'eau. Ainsi, on peut aisément stocker les composants de la composition suivant l'invention et préparer les solutions à perfuser juste avant l'administration.

## Revendications

1. Utilisation d'une composition pour la fabrication d'un médicament destiné à simultanément augmenter la quantité d'oxygène extraite au niveau des tissus et augmenter le débit cardiaque chez des mammifères atteints de troubles hypoxiques des tissus, en particulier de ceux survenant au cours de pathologies taxiinfectieuses, cette composition comprenant, en quantités thérapeutiquement efficaces,
du chlorure de sodium hypertonique,
au moins une molécule exerçant de manière directe ou indirecte un effet de relaxation des muscles lisses vasculaires, et
au moins une molécule susceptible de fournir un apport exogène en phosphates.

2. Utilisation suivant la revendication 1, **caractérisée en ce que** la composition comprend en outre un agent alcalinisant susceptible d'augmenter un pH sanguin dans une gamme compatible avec un transport d'oxygène adéquat.

3. Utilisation suivant l'une ou l'autre des revendications 1 et 2, **caractérisée en ce que** la composition se présente sous la forme d'au moins une solution aqueuse.

4. Utilisation suivant la revendication 3, **caractérisée en ce que** la composition consiste en une combinaison d'une première solution de perfusion et d'une deuxième solution de perfusion,
la première solution de perfusion comprenant de l'eau, ledit chlorure de sodium hypertonique et ladite au moins une molécule exerçant un effet de relaxation, et
la deuxième solution de perfusion comprenant de l'eau, et ladite au moins une molécule susceptible de fournir un apport exogène en phosphates.

5. Utilisation suivant l'une ou l'autre des revendications 1 et 2, **caractérisée en ce que** la composition se présente sous la forme d'au moins une formulation solide destinée à la constitution d'au moins une solution aqueuse de perfusion par mélange avec de l'eau.

6. Utilisation suivant la revendication 4, **caractérisée en ce que** le chlorure de sodium hypertonique est à une concentration de 5 à 10 % dans ladite première solution de perfusion.

7. Utilisation suivant l'une quelconque des revendications 1 à 6, **caractérisée en ce que** ladite au moins une molécule susceptible de fournir un apport exogène en phosphates est choisie parmi le groupe constitué des phosphates inorganiques, des phosphates organiques ou des modificateurs de la teneur intraérythrocytaire en diphosphoglycérate.

8. Utilisation suivant l'une quelconque des revendications 4 à 7, **caractérisée en ce que** la deuxième solution de perfusion contient en outre un agent alcalinisant susceptible d'augmenter le pH sanguin dans une gamme compatible avec un transport d'oxygène adéquat.

9. Utilisation suivant l'une quelconque des revendications 2 à 8, **caractérisée en ce que** l'agent alcalinisant est choisi parmi le groupe constitué des bicarbonate, acétates, propionates et lactates.

10. Utilisation suivant l'une quelconque des revendications 4 à 8, **caractérisée en ce que** la deuxième solution de perfusion contient en outre au moins un composant destiné à corriger l'équilibre hydrique, électrolytique et énergétique.

11. Utilisation suivant la revendication 10, **caractérisé en ce que** ledit au moins un composant destiné à corriger l'équilibre est choisi parmi le groupe constitué du chlorure de sodium, du chlorure de potassium et du glucose.

12. Utilisation suivant l'une quelconque des revendications 1 à 11, pour la mise en oeuvre d'un traitement thérapeutique ou prophylactique des troubles hypoxiques des tissus, en particulier de ceux survenant au cours de pathologies toxiinfectieuses, chez les mammifères.

13. Utilisation suivant l'une des revendications 1 à 12, dans laquelle le médicament est destiné au traitement de la diarrhée néonatale et du choc endotoxinique chez les ruminants.

14. Composition médicamenteuse pour la mise en oeuvre d'un traitement thérapeutique ou prophylactique des troubles hypoxiques des tissus, en particulier de ceux survenant au cours de pathologies toxiinfectieuses chez les mammifères, consistant en une combinaison d'une première solution de perfusion et d'une deuxième solution de perfusion,
la première solution de perfusion comprenant de l'eau, du chlorure de sodium hypertonique et au moins une molécule exerçant un effet de relaxation, et
la deuxième solution de perfusion comprenant de l'eau, et au moins une molécule susceptible de fournir un apport exogène en phosphates.

15. Composition suivant la revendication 14, **caractérisé en ce que** le chlorure de sodium hypertonique est à une concentration de 5 à 10 % dans ladite première solution de perfusion.

16. Composition suivant l'une ou l'autre des revendications 14 et 15, **caractérisée en ce que** ladite au moins une molécule susceptible de fournir un apport exogène en phosphates est choisie parmi le groupe constitué des phosphates inorganiques, des phosphates organiques ou des moduficateurs de la teneur intraérythrocytaire en diphosphoglycérate.

17. Composition suivant l'une quelconque des revendications 14 à 16, **caractérisée en ce que** la deuxième solution de perfusion contient en outre un agent alcalinisant susceptible d'augmenter le pH sanguin dans une gamme compatible avec un transport d'oxygène adéquat.

18. Composition suivant l'une quelconque des revendications 14 à 17, **caractérisée en ce que** l'agent alcalinisant est choisi parmi le groupe constitué des bicarbonates, acétates, propionates et lactates.

19. Composition suivant l'une quelconque des revendications 14 à 18, **caractérisée en ce que** la deuxième solution de perfusion contient en outre au moins un composant destiné à corriger l'équilibre hydrique, électrolytique et énergétique.

20. Composition suivant la revendication 19, **caractérisée en ce que** ledit au moins un composant destiné à corriger l'équilibre est choisi parmi le groupe constitué du chlorure de sodium, du chlorure de potassium et du glucose.

## Patentansprüche

1. Anwendung einer Zusammensetzung für die Herstellung eines Medikaments zur gleichzeitigen Erhöhung der in den Geweben extrahierten Sauerstoffmenge und des Herzminutenvolumens bei Säugetieren mit hypoxischen Gewebestörungen, insbesondere solchen, die im Laufe von toxiinfektiösen Pathologien auftreten, wobei diese Zusammensetzung gebildet wird durch - in therapeutisch effizienten Mengen -
hypertonisches Natriumchlorid,
wenigstens ein Molekül, das direkt oder indirekt einen Entspannungseffekt der glatten Gefäßmuskulatur hat, und
wenigstens ein für eine exogene Phosphatzufuhr geeignetes Molekül.

2. Anwendung nach Anspruch 1, **dadurch gekennzeichnet, dass** die Zusammensetzung außerdem einen alkalisierenden Wirkstoff umfasst, geeignet zu Erhöhung eines Blut-pH innerhalb eines Bereichs, der kompatibel ist mit einem adäquaten Sauerstofftransport.

3. Anwendung nach einem der Ansprüche 1 und 2, **dadurch gekennzeichnet, dass** die Zusammensetzung sich in Form von wenigstens einer wässrigen Lösung präsentiert.

4. Anwendung nach Anspruch 3, **dadurch gekennzeichnet, dass** die Zusammensetzung durch eine Kombination aus einer ersten Infusionslösung und einer zweiten infusionslösung gebildet wird,
wobei die erste Infusionslösung Wasser, das genannte hypertonische Natriumchlorid und das genannte wenigstens eine, einen Entspannungseffekt bewirkende Molekül umfasst,
und die zweite Infusionslösung Wasser und das genannte wenigstens eine, für eine exogene Phosphatzufuhr geeignete Molekül umfasst.

5. Anwendung nach einem der Ansprüche 1 und 2, **dadurch gekennzeichnet, dass** die Zusammensetzung sich in Form einer Feststoff-Rezeptur bzw. Trockenstoff-Formulierung präsentiert, bestimmt zur Bildung von wenigstens einer wässrigen Infusionslösung durch das Mischen mit Wasser.

6. Anwendung nach Anspruch 4, **dadurch gekennzeichnet, dass** das hypertonische Natriumchlorid in der genannten ersten Infusionslösung eine Konzentration von 5 bis 10 % hat.

7. Anwendung nach einem der Ansprüche 1 bis 6, **dadurch gekennzeichnet, dass** man wenigstens ein für eine exogene Phosphatzufuhr geeignetes Molekül aus der Gruppe auswählt, die gebildet wird durch anorganische Phosphate, organische Phosphate oder Modifikatoren des intraerythrozytären Gehalts an Diphosphoglycerat.

8. Anwendung nach einem der Ansprüche 4 bis 7, **dadurch gekennzeichnet, dass** die zweite Infusionslösung außerdem einen alkalisierenden Wirkstoff enthält, geeignet zu Erhöhung des Blut-pH innerhalb eines Bereichs, der mit einem adäquaten Sauerstofftransport kompatibel ist.

9. Anwendung nach einem der Ansprüche 2 bis 8, **dadurch gekennzeichnet, dass** man den alkalisierenden Wirkstoff aus der Gruppe auswählt, die durch Bikarbonate, Azetate, Propionate und Lactate gebildet wird.

10. Anwendung nach einem der Ansprüche 4 bis 8, **dadurch gekennzeichnet, dass** die zweite Infusionslösung außerdem wenigstens einen Bestandteil zur Korrektur des Wasser-, Elektrolyt- und Energiegleichgewichts enthält.

11. Anwendung nach Anspruch 10, **dadurch gekennzeichnet, dass** der wenigstens eine zur Korrektur des Gleichgewichts bestimmte Bestandteil ausgewählt wird aus der Gruppe, die gebildet wird durch Natriumchlorid, Kaliumchlorid und Glucose.

12. Anwendung nach einem der Ansprüche 1 bis 11, zur Durchführung einer therapeutischen oder prophylaktischen Behandlung von hypoxischen Störungen, insbesondere von solchen, die bei Säugetieren im Laufe von toxiinfektiösen Pathologien auftreten.

13. Anwendung nach einem der Ansprüche 1 bis 12, bei der das Medikament dazu bestimmt ist, den Neugeborenen-Durchfall und den septischen Schock bei Wiederkäuem zu behandeln.

14. Medikamentöse Zusammensetzung zur Durchführung einer therapeutischen oder prophylaktischen Behandlung von hypoxischen Gewebestörungen, insbesondere solchen, die bei Säugetieren im Laufe von toxiinfektiösen Pathologien auftreten, gebildet durch eine Kombination aus einer ersten Infusionslösung und einer zweiten Infusionslösung,
wobei die erste Infusionslösung Wasser, hypertonisches Natriumchlorid und wenigstens ein einen Entspannungseffekt bewirkendes Molekül umfasst,
und die zweite Infusionslösung Wasser und wenigstens ein für eine exogene Phosphatzufuhr geeignetes Molekül umfasst.

15. Zusammensetzung nach Anspruch 14, **dadurch gekennzeichnet, dass** das hypertonische Natriumchlorid in der genannten ersten Infusionslösung eine Konzentration von 5 bis 10 % hat.

16. Zusammensetzung nach einem der Ansprüche 14 und 15, **dadurch gekennzeichnet, dass** man das genannte wenigstens eine für eine exogene Phosphatzufuhr geeignete Molekül aus der Gruppe auswählt, die gebildet wird durch anorganische Phosphate, organische Phosphate oder Modifikatoren des intraerythrozytären Gehalts an Diphosphoglycerat.

17. Zusammensetzung nach einem der Ansprüche 14 bis 16, **dadurch gekennzeichnet, dass** die zweite Infusionslösung außerdem
einen alkalisierenden Wirkstoff umfasst, geeignet zu Erhöhung des Blut-pH innerhalb eines Bereichs, der mit einem adäquaten Sauerstofftransport kompatibel ist.

18. Zusammensetzung nach einem der Ansprüche 14 bis 17, **dadurch gekennzeichnet, dass** man den alkalisierenden Wirkstoff aus der Gruppe auswählt, die durch Bikarbonate, Azetate, Propionate und Lactate gebildet wird.

19. Zusammensetzung nach einem der Ansprüche 14 bis 18, **dadurch gekennzeichnet, dass** die zweite Infusionslösung außerdem wenigstens einen Bestandteil zur Korrektur des Wasser-, Elektrolyt- und Energiegleichgewichts enthält.

20. Anwendung nach Anspruch 19, **dadurch gekennzeichnet, dass** der genannte wenigstens eine zur Korrektur des Gleichgewichts bestimmte Bestandteil ausgewählt wird aus der Gruppe, die durch Natriumchlorid, Kaliumchlorid und Glucose gebildet wird.

## Claims

1. Use of a composition for manufacturing a medicine intended to simultaneously increase the quantity of oxygen extracted at the tissues and increase the cardiac output in mammals suffering from hypoxic tissue disorders, in particular those arising during toxi-infectious pathologies, this composition comprising, in therapeutically effective quantities,
hypertonic sodium chloride,
at least one molecule directly or indirectly exerting a relaxation effect on the vascular smooth muscles, and
at least one molecule able to supply an exogenous phosphate input.

2. Use according to Claim 1, **characterised in that** it also comprises an alkalinising agent able to increase the blood pH in a range compatible with suitable transportation of oxygen.

3. Use according to one or other of Claims 1 and 2, **characterised in that** the composition is in the form of at least one aqueous solution.

4. Use according to Claim 3, **characterised in that** it consists of a combination of a first perfusion solution and a second perfusion solution,
the first perfusion solution comprising water, the said hypertonic sodium chloride and the said at least one molecule exerting a relaxation effect, and
the second perfusion solution comprising water, and the said at least one molecule able to supply an exogenous phosphate input.

5. Use according to one or other of Claims 1 and 2, **characterised in that** the composition is in the form of at least one solid formulation intended for constituting at least one aqueous perfusion solution by mixing with water.

6. Use according to Claim 4, **characterised in that** the hypertonic sodium chloride is at a concentration of 5% to 10% in the said first perfusion solution.

7. Use according to any one of Claims 1 to 6, **characterised in that** the said at least one molecule able to supply an exogenous phosphate input is chosen from amongst the group consisting of inorganic phosphates, organic phosphates or modifiers of the intra-erythrocyte diphosphoglycerate content.

8. Use according to any one of Claims 4 to 7, **characterised in that** the second perfusion solution also contains an alkalinising agent able to increase the blood pH in a range compatible with a suitable transportation of oxygen.

9. Use according to any one of Claims 2 to 8, **characterised in that** the alkalinising agent is chosen from amongst the group consisting of bicarbonates, acetates, propionates and lactates.

10. Use according to any one of Claims 4 to 8, **characterised in that** the second perfusion solution also contains at least one component intended to correct the water, electrolytic or energy balance.

11. Use according to Claim 10, **characterised in that** the said at least one component intended to correct the balance is chosen from amongst the group consisting of sodium chloride, potassium chloride and glucose.

12. Use according to any one of Claims 1 to 11 for implementing a therapeutic or prophylactic treatment for hypoxic tissue disorders, in particular those arising during toxi-infectious pathologies, in mammals.

13. Use according to Claim 12 wherein the medicine is intended in the treatment of neonatal diarrhoea and endotoxin shock in ruminants.

14. Medicinal composition to be used in a therapeutic or prophylactic treatment of hypoxic tissue disorders, in particular those arising during toxi-infectious pathologies, in mammals, consisting of a combination of a first perfusion solution and a second perfusion solution,
the first perfusion solution comprising water, the said hypertonic sodium chloride and the said at least one molecule exerting a relaxation effect, and
the second perfusion solution comprising water, and the said at least one molecule able to supply an exogenous phosphate input.

15. Composition according to Claim 14, **characterised in that** the hypertonic sodium chloride is at a concentration of 5% to 10% in the said first perfusion solution.

16. Composition according to either one of Claims 14 and 15, **characterised in that** the said at least one molecule able to supply an exogenous phosphate input is chosen from amongst the group consisting of inorganic phosphates, organic phosphates or modifiers of the intra-erythrocyte diphosphoglycerate content.

17. Composition according to any one of Claims 14 to 16, **characterised in that** the second perfusion solution also contains an alkalinising agent able to increase the blood pH in a range compatible with a suitable transportation of oxygen.

18. Composition according to any one of Claims 14 to 17, **characterised in that** the alkalinising agent is chosen from amongst the group consisting of bicarbonates, acetates, propionates and lactates.

19. Compostion according to any one of Claims 14 to 18, **characterised in that** the second perfusion solution also contains at least one component intended to correct the water, electrolytic or energy balance.

20. Composition according to Claim 19, **characterised in that** the said at least one component intended to correct the balance is chosen from amongst the group consisting of sodium chloride, potassium chloride and glucose.
